# EUROPEAN PATENT SPECIFICATION

(11) **EP 2 952 190 B1**
(45) Date of publication and mention of the grant of the patent: **06.09.2017**
(21) Application number: 14745605.7
(22) Date of filing: 30.01.2014
(51) Int. Cl.: A61K 9/08, A61K 47/10, A61P 27/02, A61K 31/427

(54) **STABLE AQUEOUS SOLUTION**
STABILE WÄSSRIGE LÖSUNG
SOLUTION AQUEUSE STABLE

(30) Priority: 31.01.2013 JP 2013017877; 21.08.2013 JP 2013171433
(43) Date of publication of application: 09.12.2015
(73) Proprietor: SENJU PHARMACEUTICAL CO., LTD., Osaka-shi Osaka 541-0046 (JP)
(72) Inventor: SHIKAMURA, Yuko, Kobe-shi Hyogo 651-2241 (JP); HIGASHIMURA, Yuka, Kobe-shi Hyogo 651-2241 (JP)
(74) Representative: Webster, Jeremy Mark
(86) International application number: PCT/JP2014/052040
(87) International publication number: WO 2014/119642

(56) References cited:
- EP-A1- 2 952 189
- WO-A1-02/067977
- WO-A1-2004/064828
- WO-A1-2008/143254
- JP-A- H02 286 627
- JP-A- H11 228 404
- JP-A- 2005 247 800
- JP-A- 2010 540 682

## Description

### Technical Field

The present invention relates to a method of stabilizing (3-{2-[4-isopropyl-2-(4-trifluoromethyl)phenyl-5-thiazolyl]ethyl}-5-methyl-1,2-benzisoxazol-6-yl)oxyacetic acid or a pharmaceutically acceptable salt thereof, and a stabilized aqueous liquid preparation containing (3-{2-[4-isopropyl-2-(4-trifluoromethyl)phenyl-5-thiazolyl]ethyl}-5-methyl-1,2-benzisoxazol-6-yl)oxyacetic acid.

### Background Art

It has been reported that (3-{2-[4-isopropyl-2-(4-trifluoromethyl)phenyl-5-thiazolyl]ethyl}-5-methyl-1,2-benzisoxazol-6-yl)oxyacetic acid is a medicament having a Peroxisome Proliferator-Activated Receptor (hereinafter to be referred to as PPAR) δ agonist action (see patent document 1). In addition, it has been reported that (3-{2-[4-isopropyl-2-(4-trifluoromethyl)phenyl-5-thiazolyl]ethyl}-5-methyl-1,2-benzisoxazol-6-yl)oxyacetic acid or a pharmaceutically acceptable salt thereof (hereinafter sometimes to be referred to as compound A) is a PPARδ agonist and useful as a proliferation promoter of meibomian gland epithelial cell or corneal epithelial cell (see patent document 2).

The present inventors have confirmed in a development process of an aqueous liquid preparation containing compound A that compound A is practically insoluble in water, extremely unstable to light in an aqueous solution, and becomes unstable to heat depending on the combination with additive. This has already been shown by the fact that the heat stability of compound A in an aqueous solution decreases extremely when polysorbate 80 conventionally widely used as a solubilizer of poorly soluble drugs is added (see below-mentioned Experimental Example 2, Comparative Example 1).

To sufficiently utilize a superior pharmacological effect of an aqueous liquid preparation containing compound A, it is highly important to secure its water-solubility, and stability to light and heat during production steps and distribution processes, during use, and for a long term after opening. However, there is no report on stability and stabilization of compound A to light and heat.

Generally, since stabilization of a drug is influenced not only by the physical properties and chemical properties intrinsic to the drug, but also the properties of the drug, the physical properties and chemical properties of the additives to be used, a combination method thereof and the like, it is not easy to generalize the preparation method thereof.

### [Document List]

### [patent documents]

patent document 1: WO 03/033493
patent document 2: WO 2008/143254

### SUMMARY OF THE INVENTION

### Problems to be Solved by the Invention

The present invention aims to provide an aqueous liquid preparation containing (3-{2-[4-isopropyl-2-(4-trifluoromethyl)phenyl-5-thiazolyl]ethyl}-5-methyl-1,2-benzisoxazol-6-yl)oxyacetic acid and having high stability to light and heat.

### Means of Solving the Problems

The present inventors have conducted intensive studies in an attempt to solve the aforementioned problems and found that the stability to light and heat of compound A in a composition containing compound A as an active ingredient is remarkably improved by adding tyloxapol or octoxynol to the composition. They have also found that the stability of compound A to heat is additionally improved by adding alcohol (e.g., glycerin, sugar alcohol, glycol, ethanol etc.) in addition to tyloxapol or octoxynol, which resulted in the completion of the present invention.

Accordingly, the present invention relates to
[1] an aqueous liquid preparation comprising (3-{2-[4-isopropyl-2-(4-trifluoromethyl)phenyl-5-thiazolyl]ethyl}-5-methyl-1,2-benzisoxazol-6-yl)oxyacetic acid or a pharmaceutically acceptable salt thereof, and tyloxapol or octoxynol,
[2] an aqueous liquid preparation comprising (3-{2-[4-isopropyl-2-(4-trifluoromethyl)phenyl-5-thiazolyl]ethyl}-5-methyl-1,2-benzisoxazol-6-yl)oxyacetic acid or a pharmaceutically acceptable salt thereof, and tyloxapol,
[3] an aqueous liquid preparation comprising (3-{2-[4-isopropyl-2-(4-trifluoromethyl)phenyl-5-thiazolyl]ethyl}-5-methyl-1,2-benzisoxazol-6-yl)oxyacetic acid or a pharmaceutically acceptable salt thereof, and octoxynol,
[4] the aqueous liquid preparation of any of the above-mentioned [1] - [3], wherein (3-{2-[4-isopropyl-2-(4-trifluoromethyl)phenyl-5-thiazolyl]ethyl}-5-methyl-1,2-benzisoxazol-6-yl)oxyacetic acid or a pharmaceutically acceptable salt thereof has a concentration selected from the range of the lower limit concentration of 0.0002 w/v% and the upper limit concentration of 0.05 w/v% relative to the total amount of the aqueous liquid preparation,
[5] the aqueous liquid preparation of the above-mentioned [1], [2] or [4], wherein tyloxapol has a concentration selected from the range of the lower limit concentration of 0.01 w/v% and the upper limit concentration of 0.5 w/v% relative to the total amount of the aqueous liquid preparation,
[6] the aqueous liquid preparation of the above-mentioned [1], [3] or [4], wherein octoxynol has a concentration selected from the range of the lower limit concentration of 0.01 w/v% and the upper limit concentration of 0.5 w/v% relative to the total amount of the aqueous liquid preparation,
[7] the aqueous liquid preparation of any of the above-mentioned [1] - [6], further comprising alcohol,
[8] the aqueous liquid preparation of the above-mentioned [7], wherein alcohol has a concentration selected from the range of the lower limit concentration of 0.1 w/v% and the upper limit concentration of 5 w/v% relative to the total amount of the aqueous liquid preparation,
[9] the aqueous liquid preparation of the above-mentioned [7] or [8], wherein alcohol comprises at least any selected from glycerin, sugar alcohol, glycol and ethanol,
[10] the aqueous liquid preparation of the above-mentioned [7] or [8], wherein alcohol comprises at least any selected from glycerin, mannitol, propylene glycol and ethanol,
[11] the aqueous liquid preparation of the above-mentioned [7] or [8], wherein alcohol comprises propylene glycol,
[12] the aqueous liquid preparation of any of the above-mentioned [1] - [11], which has transmittance at wavelength 600 nm of not less than 98%,
[13] the aqueous liquid preparation of any of the above-mentioned [1] - [12], which is for ophthalmology,
[14] the aqueous liquid preparation of the above-mentioned [13], which is an ophthalmic solution,
[15] use of tyloxapol or octoxynol in an aqueous liquid preparation of any of the above-mentioned [1] to [12] comprising (3-{2-[4-isopropyl-2-(4-trifluoromethyl)phenyl-5-thiazolyl]ethyl}-5-methyl-1,2-benzisoxazol-6-yl)oxyacetic acid or a pharmaceutically acceptable salt thereof, for stabilizing the (3-{2-[4-isopropyl-2-(4-trifluoromethyl)phenyl-5-thiazolyl]ethyl}-5-methyl-1,2-benzisoxazol-6-yl)oxyacetic acid or a pharmaceutically acceptable salt thereof in the aqueous liquid preparation to light and heat,
[16] a method of stabilizing (3-{2-[4-isopropyl-2-(4-trifluoromethyl)phenyl-5-thiazolyl]ethyl}-5-methyl-1,2-benzisoxazol-6-yl)oxyacetic acid or a pharmaceutically acceptable salt thereof to light and heat in an aqueous liquid preparation, comprising adding tyloxapol or octoxynol to an aqueous liquid preparation comprising (3-{2-[4-isopropyl-2-(4-trifluoromethyl)phenyl-5-thiazolyl]ethyl}-5-methyl-1,2-benzisoxazol-6-yl)oxyacetic acid or a pharmaceutically acceptable salt thereof, thereby to produce an aqueous liquid preparation in accordance with any of the above-mentioned [1] to [12].

### Effect of the Invention

According to the present invention, an aqueous liquid preparation containing compound A useful as a therapeutic agent for ocular diseases such as meibomian gland dysfunction, corneal epithelial disorder, dry eye and the like, and having high stability to light and heat can be provided. In addition, addition of tyloxapol, octoxynol has an effect of improving stability of an aqueous solution containing compound A to light and heat, and also improving solubility of compound A. Description of Embodiments

The present invention is further explained in detail in the following.

In the present specification, unless particularly indicated, w/v% means weight per volume percentage in the Japanese Pharmacopoeia, 16th Edition. Unless particularly indicated, the contact lens encompasses any type of contact lens such as hard, oxygen permeable hard, soft and the like.

Since the aqueous liquid preparation of the present invention is useful as a proliferation promoter of meibomian gland epithelial cell or corneal epithelial cell due to the PPARδ agonist action of compound A, it can be used for the treatment of ocular diseases such as meibomian gland dysfunction, corneal epithelial disorder, dry eye and the like.

Compound A used in the present invention includes any pharmaceutically acceptable salt. Examples of the pharmaceutically acceptable salt include, but are not limited to, salts with inorganic base such as sodium, potassium, calcium, magnesium, aluminum and the like, ammonium salt, salts with organic base such as methylamine, triethylamine, diethylamine, morpholine, piperazine, pyrrolidine, picoline, ethanolamine, lysine, arginine and the like. Compound A can be produced according to the method described in WO 03/033493.

In the aqueous liquid preparation of the present invention, the ratio of compound A to be added is not particularly limited as long as the effect of the present invention can be afforded. For example, the lower limit is generally about 0.00001 w/v%, preferably about 0.0001 w/v%, more preferably about 0.0002 w/v%, particularly preferably about 0.001 w/v%, most preferably about 0.005 w/v%, and the upper limit is generally about 1 w/v%, preferably about 0.1 w/v%, more preferably about 0.05 w/v%, particularly preferably about 0.01 w/v%, relative to the total amount of the aqueous liquid preparation.

The aqueous liquid preparation of the present invention can be prepared by adding tyloxapol or octoxynol to compound A. Furthermore, a compound A-containing aqueous liquid preparation stable to light and heat can be provided by adding alcohol as necessary.

Tyloxapol is a compound represented by the following formula:

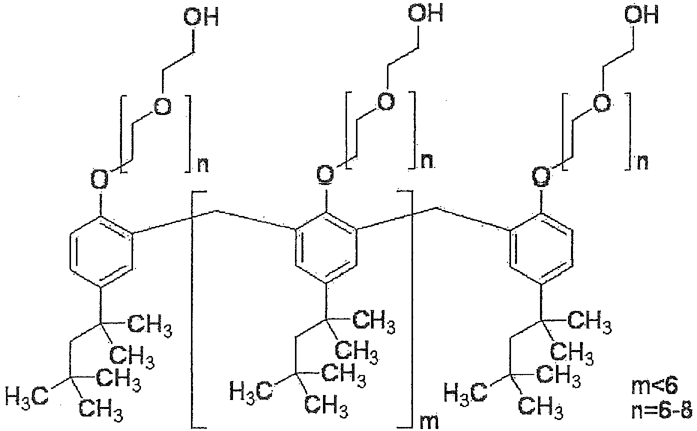

It is sometimes referred to as Formaldehyde, polymer with oxirane and 4-(1,1,3,3-tetramethylbutyl)phenol, Superinone. For example, it is available as Tyloxapol USP from Ruger Chemical Co., Inc.

Octoxynol is a compound represented by the following formula:

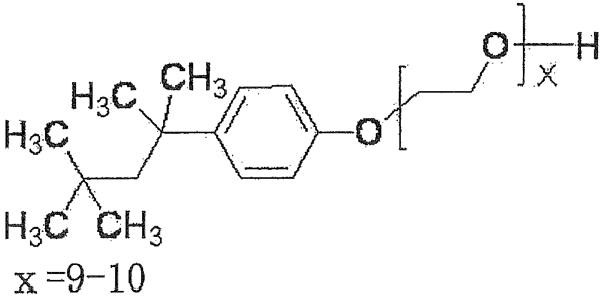

It is sometimes referred to as a-[4-(1,1,3,3-tetramethylbutyl)phenyl]-ω-hydroxypoly(oxy-1,2-ethanediyl), polyethylene glycol p-isoctylphenyl ether, Triton X 100. For example, it is available as Triton(R)X-100 from Nacalai Tesque.

Of these, from the aspect of use for application as an ophthalmic solution, tyloxapol is particularly preferable.

The amount of tyloxapol to be used for the aqueous liquid preparation of the present invention may be appropriately determined according to the amount of compound A to be added. The lower limit of tyloxapol is generally about 0.001 w/v%, preferably about 0.01 w/v%, more preferably about 0.05 w/v%, and the upper limit is generally about 1.0 w/v%, preferably about 0.5 w/v%, more preferably about 0.2 w/v%, particularly preferably about 0.1 w/v%, relative to the total amount of the aqueous liquid preparation.

The aqueous liquid preparation of the present invention can use, besides tyloxapol, other conventional surfactants usable for ophthalmic application in an appropriate combination, as long as the stability of compound A is not impaired.

The amount of octoxynol to be used for the aqueous liquid preparation of the present invention may be appropriately determined according to the amount of compound A to be used. The lower limit of octoxynol is generally about 0.001 w/v%, preferably about 0.01 w/v%, more preferably about 0.05 w/v%, and the upper limit is generally about 1.0 w/v%, preferably about 0.5 w/v%, more preferably about 0.2 w/v%, particularly preferably about 0.1 w/v%, relative to the total amount of the aqueous liquid preparation.

The aqueous liquid preparation of the present invention can use, besides octoxynol, other conventional surfactants usable for ophthalmic application in an appropriate combination, as long as the stability of compound A is not impaired.

Examples of alcohol to be used for the aqueous liquid preparation of the present invention include polyhydric alcohols such as glycerin and the like; sugar alcohols such as mannitol, sorbitol and the like; glycols (dihydric alcohol) such as ethylene glycol, propylene glycol, trimethylene glycol, 1,2-butylene glycol, 1,3-butylene glycol, 2,3-butylene glycol, isoprene glycol, 1,2-pentylene glycol, 1,2-hexylene glycol, octylene glycol, diethylene glycol, triethylene glycol, dipropylene glycol, polyethylene glycol and the like; monohydric alcohols such as ethanol and the like, and the like. Preferred is glycerin, mannitol or propylene glycol, particularly preferred is propylene glycol. One kind of these alcohols may be used alone, or two or more kinds thereof may be used in an appropriate combination. The amount of alcohol to be added can be appropriately determined according to the kind of alcohol, amount of compound A, tyloxapol and octoxynol to be added and the like. The lower limit is generally about 0.01 w/v%, preferably about 0.1 w/v%, more preferably about 0.5 w/v%, and the upper limit is generally about 10 w/v%, preferably about 5 w/v%, relative to the total amount of the aqueous liquid preparation.

A preferable embodiment of the aqueous liquid preparation of the present invention is an aqueous liquid preparation containing compound A, tyloxapol and propylene glycol.

Another preferable embodiment of the aqueous liquid preparation of the present invention is an aqueous liquid preparation containing compound A, octoxynol and propylene glycol.

An appropriate pH of the aqueous liquid preparation of the present invention varies depending on the application site, dosage form and the like. For use as an ophthalmic solution, it is generally about 6.0 - about 8.6.

The pH can be adjusted using the below-mentioned buffering agent, pH adjuster and the like and according to a method known in the pertinent technical field. In addition, the kinds of the buffering agent, pH adjuster and the like to be used do not influence the stability of compound A in the aqueous liquid preparation.

In the present specification, being stable to light and heat means that degradation of compound A by light and heat is suppressed in an aqueous solution containing compound A. To be specific, when an aqueous solution containing compound A is filled in a colorless glass ampoule and stored, being stable to light means level of degradation products after light irradiation at 12000 lux·h or 24000 lux·h is suppressed as compared to Reference Examples free of tyloxapol or octoxynol, that is, the residual ratio of compound A is higher. Also, being stable to heat means that the residual ratio of compound A after storage at 80°C for 1 week is not less than 70%, the residual ratio of compound A is preferably not less than 80%, more preferably not less than 90%. A particularly preferable embodiment stable to heat is when the residual ratio of compound A after storage at 80°C for 1 week is higher than Reference Examples free of tyloxapol or octoxynol.

Various additives such as buffering agent, isotonicity agent, preservative, solubilizing agent, stabilizer, chelating agent, cooling agent, thickener, pH adjuster and the like can be added as necessary to the aqueous liquid preparation of the present invention.

Examples of the buffering agent include known boric acid buffers (borax etc.), citrate buffer (sodium citrate etc.), carbonate buffer (sodium hydrogen carbonate, sodium carbonate etc.), tartrate buffer (sodium tartrate etc.), gluconate buffer (sodium gluconate etc.), acetate buffer (sodium acetate etc.), phosphate buffer (sodium monohydrogen phosphate, sodium dihydrogen phosphate etc.), various amino acids such as glutamic acid, epsilon aminocaproic acid and the like, Tris buffer, Good buffer (MES, MOPS, PIPES, HEPES, BES, TES etc.) and the like, or a combination thereof.

Examples of the isotonicity agent include polyhydric alcohols such as sorbitol, glucose, mannitol, glycerin, propylene glycol and the like, salts such as sodium chloride, potassium chloride and the like, boric acid and the like.

Examples of the preservative include paraoxybenzoates, benzalkonium chloride, benzethonium chloride, benzyl alcohol, sorbic acid or a salt thereof, chlorhexidine gluconate, sodium dehydroacetate, cetyl pyridinium chloride, alkyldiaminoethylglycine hydrochloride, chlorobutanol, thimerosal and the like.

Examples of the solubilizing agent include polyvinylpyrrolidone, polyethylene glycol, propylene glycol, sodium carboxymethylcellulose, glycerin and the like.

Examples of the stabilizer include sodium edetate, sodium thiosulfate, thioglycolic acid, sodium thioglycolate, cysteine hydrochloride, ascorbic acid, cyclodextrin, condensed phosphoric acid or a salt thereof, sulfite, citric acid or a salt thereof, dibutylhydroxytoluene and the like.

Examples of the chelating agent include sodium edetate, sodium citrate, thioglycolic acid, sodium thioglycolate, thiolactic acid, thioglycerin, condensed phosphoric acid or a salt thereof (condensed sodium phosphate etc.) and the like.

Examples of the thickener include methylcellulose, hydroxyethylcellulose, hydroxypropylcellulose, hydroxypropylmethylcellulose, sodium chondroitin sulfate, sodium carboxymethylcellulose, polyvinylpyrrolidone, polyvinyl alcohol, polyethylene glycol and the like.

Examples of the pH adjuster include sodium hydroxide, potassium hydroxide, sodium carbonate, sodium hydrogen carbonate, boric acid or a salt thereof (borax), hydrochloric acid, citric acid or a salt thereof (sodium citrate, sodium dihydrogen citrate etc.), phosphoric acid or a salt thereof (disodium hydrogen phosphate, potassium dihydrogen phosphate etc.), acetic acid or a salt thereof (sodium acetate, ammonium acetate etc.), tartaric acid or a salt thereof (sodium tartrate etc.), amines such as monoethanolamine, diethanolamine, triethanolamine, meglumine and the like, and the like.

Examples of the cooling agent include menthol, borneol, camphor, mentha oil, eucalyptus oil, peppermint oil and the like. These may be any of d form, 1 form and dl form.

When a suspension is prepared, usable as the suspending agent are methylcellulose, sodium carboxymethylcellulose, carboxyvinyl polymer, hydroxypropylmethylcellulose, hydroxymethylcellulose, polyvinyl alcohol, polyvinylpyrrolidone, polyethylene glycol, polysorbate 80, aluminum monostearate and the like.

When an emulsion is prepared, for example, vegetable oils such as castor oil, olive oil, sesame oil, soybean oil, camellia oil, rape seed oil, corn oil, peanut oil, cotton seed oil and the like, animal oils such as squalane and the like, as well as liquid paraffin and the like can be used as an oil. As an emulsifier, surfactants such as polyoxyethylene sorbitan monolaurate, sorbitan ester of fatty acid, polyoxyethylene sorbitol beeswax, polyethylene glycol monostearate, polyoxyethylene hydrogenated castor oil, polysorbate 80 and the like, polyvinylpyrrolidone, purified egg-yolk lecithin, soybean lecithin and the like can be used.

The aqueous liquid preparation to be used in the present invention can be used, for example, as ophthalmic solution, eye wash, agents for contact lenses, injection and the like, and an ophthalmic solution to be topically instilled into the eye is particularly preferable. Preferable examples of the administration method include, but are not particularly limited to, dropwise administration such as instillation and the like, eye wash using an eye wash cup and the like.

The aforementioned agents for contact lenses can be applied to any contact lenses including hard contact lenses and soft contact lenses.

Examples of the form of the ophthalmic solution of the present invention include aqueous solution, suspension, emulsion and the like, with preference given to an aqueous solution.

The aqueous liquid preparation of the present invention is preferably a clear aqueous liquid preparation. Here, an aqueous liquid preparation being "clear" means, unless particularly indicated, a state where light transmittance at wavelength 600 nm is not less than 98.0%, which is not limited to colorless clear but also includes colored clear due to other components contained therein. A state of transmittance being less than 98.0% is taken as a clouded state.

The ophthalmic solution of the present invention is produced according to a preparation method known per se (e.g., the method described in the Japanese Pharmacopoeia, 16th Edition, Preparation General Rules, section of ophthalmic liquids and solutions, and the like). For example, the ophthalmic solution of the present invention can be produced by dissolving other additives such as solubilizer, buffering agent, isotonicity agent, preservative and the like in distilled water or purified water, then dissolving compound A, adding alcohol, adjusting the osmotic pressure and pH to predetermined levels, and sterilizing the mixture by filtration and aseptically filling same in a washed and sterilized container under aseptic environment.

When formulated as an ophthalmic solution, the aqueous liquid preparation is preferably contained in an instillation container provided with a liquid injection pore having a small diameter that can control droplet amount to facilitate dropping to the eyes. While the material of the container is not particularly limited, a container having low moisture permeability, a container to which respective components do not easily adsorb, a container having high transparency and the like are preferable. Specifically, for example, as the material of the container, synthetic resin, glass, cellulose, pulp and the like are used. From the aspects of squeezability and durability, the container is preferably made of a synthetic resin. Specific examples of the synthetic resin include polyethylene resin (e.g., low density polyethylene or high density polyethylene), polypropylene resin, ethylene-propylene copolymer resin, poly(ethylene terephthalate) resin, polycarbonate resin and the like.

Examples of the instillation container include a container wherein a spigot member and a container body, which are independently molded, are fit into an integrally-molded container wherein a liquid is tightly sealed simultaneously with the molding of the container (e.g., WO 2004/006826) and the like. When an integrally-molded container is employed, the container is superior in the aspect of cost or hygiene, since the container and the aqueous liquid preparation are continuously produced. The instillation container may be a unit dose type container to be disposed after each time of use (e.g., JP-A-9-207959). When this container is employed, a preparation free of a preservative, which is highly safe to the cornea, can be formulated. In addition, these containers may be adhesion-packed with a UV blocking film. Furthermore, the containers may be colored to enhance the UV blocking performance. The ophthalmic solution of the present invention has improved stability to light, and therefore, any of a light transparent container and a light semi-transparent container can be used preferably.

When the aqueous liquid preparation of the present invention is used as an ophthalmic solution, it is generally administered by adding dropwise, spraying or applying 1 - 2 drops, i.e., about 50 - 200 µL per instillation, to one eye 1 - 8 times per day, though subject to variation depending on the age, body weight and conditions of the subject of administration, treatment object, administration form and the like. When used as an eye wash, several mL thereof is used for washing one time, and washing is performed once to several times per day.

### Examples

While the present invention is explained in detail by referring to the following Experimental Examples and Formulation Examples, they do not limit the present invention. In the following Experimental Examples and Formulation Examples, (3-{2-[4-isopropyl-2-(4-trifluoromethyl)phenyl-5-thiazolyl]ethyl}-5-methyl-1,2-benzisoxazol-6-yl)oxyacetic acid was used as compound A. All preparations of the following Examples and Formulation Examples are clear aqueous liquid preparations having a light transmittance at wavelength 600 nm of not less than 98.0%.

In the following Experimental Examples, HCO-60 is polyoxyethylene hydrogenated castor oil 60 and MYS-40 is polyoxyl 40 stearate.

[Experimental Example 1] Light stability of compound A in aqueous solution (the addition of polysorbate 80, HCO-60 or MYS-40 is not according to the invention)

### (Test method)

An aqueous solution of compound A was prepared according to the following formulation. In 0.1% phosphate buffer was added a predetermined amount of tyloxapol, octoxynol, polysorbate 80, HCO-60 or MYS-40, and the mixture was adjusted to pH 7.5 with sodium hydroxide. Compound A in the predetermined amount in the following formulation was dissolved in said solution, and the mixture was sterilized by filtration with a 0.22 µm membrane filter, and filled in a 5 mL colorless glass ampoule. Using a photostability testing device (model: LT-120A-WCD, manufactured by Nagano Science Co., Ltd.), the ampoule was exposed to white light (total illumination 12000 lux·h and 24000 lux·h), and the content of compound A in the glass ampoule was measured.

Compound A after storage was quantified by high performance liquid chromatography using the absolute calibration curve method (the Japanese Pharmacopoeia).

### High-performance liquid chromatography conditions

detector: ultraviolet absorption spectrophotometer (measurement wavelength: 313 nm)
column: stainless tube (inner diameter 4.6 mm, length 25 cm) packed with 5 µm phenylated silica gel for high performance liquid chromatography (COSMOSIL 5PE-MS Packed Column, Nacalai Tesque).
column temperature: constant temperature near 30°C mobile phase A: 5 mM phosphate buffer (pH 6.0)/acetonitrile mixed solution (60:40)
mobile phase B: 5 mM phosphate buffer (pH 6.0)/acetonitrile/methanol mixed solution (20:40:40) flow rate: about 1.4 mL/min
gradient elution conditions:

**Table 1**

| time (min) | mobile phase A (%) | mobile phase B (%) |
|---|---|---|
| 0-13 | 100 | 0 |
| 13-16 | 100→0 | 0→100 |
| 16-26 | 0 | 100 |
| 26-27 | 0→100 | 100→0 |
| 27-40 | 100 | 0 |

**[Table 2-1]**

| component* | content (w/v%) | | | | | |
|---|---|---|---|---|---|---|
| | Ref. Ex. | Ex. 1 | Ex. 2 | Comp. Ex. 1 | Comp. Ex. 2 | Comp. Ex. 3 |
| Compound A | 0.0005 | 0.0005 | 0.0005 | 0.0005 | 0.0005 | 0.0005 |
| tyloxapol | - | 0.10 | - | - | - | - |
| octoxynol | - | - | 0.10 | - | - | - |
| polysorbate80 | - | - | - | 0.10 | - | - |
| HCO-60 | - | - | - | - | 0.10 | - |
| MYS-40 | - | - | - | - | - | 0.10 |
| pH | 7.5 | 7.5 | 7.5 | 7.5 | 7.5 | 7.5 |
| residual ratio (%) after light irradiation (12000 lux·h) | 70.3 | 94.2 | 92.6 | 89.5 | 79.1 | 81.2 |
| residual ratio (%) after light irradiation (24000 lux·h) | 46.0 | 89.2 | 86.9 | 80.6 | 67.1 | 66.8 |

| | | | | | | |
|---|---|---|---|---|---|---|
| *: sodium dihydrogen phosphate (0.1 w/v%), sodium hydroxide/hydrochloric acid (q.s.) and purified water (q.s.) were contained as other components | | | | | | |

**[Table 2-2]**

| component* | content (w/v%) | | | | | | |
|---|---|---|---|---|---|---|---|
| | Ex. 3 | Ex. 4 | Ex. 5 | Ex. 6 | Ex. 7 | Ex. 8 | Ex. 9 |
| Compound A | 0.0002 | 0.0010 | 0.005 | 0.05 | 0.0005 | 0.0005 | 0.0005 |
| tyloxapol | 0.10 | 0.10 | 0.10 | 0.50 | 0.01 | 0.05 | 0.50 |
| pH | 7.5 | 7.5 | 7.5 | 7.5 | 7.5 | 7.5 | 7.5 |
| residual ratio (%) after light irradiation (12000 lux· h) | 91.2 | 95.1 | 97.0 | 99.2 | 86.2 | 92.5 | 94.1 |
| residual ratio (%) after light irradiation (24000 lux· h) | 84.5 | 90.3 | 94.8 | 98.9 | 76.2 | 84.1 | 88.6 |

| | | | | | | | |
|---|---|---|---|---|---|---|---|
| *: sodium dihydrogen phosphate (0.1 w/v%), sodium hydroxide/hydrochloric acid (q.s.) and purified water (q.s.) were contained as other components | | | | | | | |

### (Test results)

In Reference Example, compound A in the preparation was remarkably unstable to light. In contrast, compound A in the preparation could be stabilized to light (Examples 1 and 2 of Table 2-1 and Examples 3 - 9 of Table 2-2), as compared to other preparations (Comparative Example 1, Comparative Example 2 or Comparative Example 3 of Table 2-1), by adding tyloxapol or octoxynol.

[Experimental Example 2] Heat stability of compound A in aqueous solution (the addition of polysorbate 80, HCO-60 or MYS-40 is not according to the invention)

### (Test method)

An aqueous solution of compound A was prepared according to the following formulation. In 0.1% phosphate buffer was added a predetermined amount of tyloxapol, octoxynol, polysorbate 80, HCO-60 or MYS-40, and the mixture was adjusted to pH 7.5 with sodium hydroxide. Compound A in the predetermined amount in the following formulation was dissolved in said solution, and the mixture was sterilized by filtration with a 0.22 µm membrane filter, and filled in a 5 mL colorless glass ampoule. The glass ampoule was stored at 80°C for 1 week, and the content of compound A after storage was measured.

Compound A after storage was quantified by high performance liquid chromatography using the absolute calibration curve method (the Japanese Pharmacopoeia).

### High-performance liquid chromatography conditions

detector: ultraviolet absorption spectrophotometer (measurement wavelength: 313 nm)
column: stainless tube (inner diameter 4.6 mm, length 25 cm) packed with 5 µm octadecylsilylated silica gel for high performance liquid chromatography (L-column ODS, Chemicals Evaluation and Research Organization).
column temperature: constant temperature near 25°C
mobile phase: acetonitrile/0.1% phosphoric acid mixed solution (4:1)
flow rate: about 1 mL/min

**[Table 3-1]**

| component* | content (w/v%) | | | | | |
|---|---|---|---|---|---|---|
| | Ref. Ex. | Ex. 1 | Ex. 2 | Comp. Ex. 1 | Comp. Ex. 2 | Comp. Ex. 3 |
| Compound A | 0.0005 | 0.0005 | 0.0005 | 0.0005 | 0.0005 | 0.0005 |
| tyloxapol | - | 0.10 | - | - | - | - |
| octoxynol | - | - | 0.10 | - | - | - |
| polysorbate 80 | - | - | - | 0.10 | - | - |
| HCO-60 | - | - | - | - | 0.10 | - |
| MYS-40 | - | - | - | - | - | 0.10 |
| pH | 7.5 | 7.5 | 7.5 | 7.5 | 7.5 | 7.5 |
| residual ratio (%) of compound A after storage at 80°C for 1 week | 98.5 | 90.3 | 95.2 | 21.2 | 30.6 | 28.2 |

| | | | | | | |
|---|---|---|---|---|---|---|
| *: sodium dihydrogen phosphate (0.1 w/v%), sodium hydroxide/hydrochloric acid (q.s.) and purified water (q.s.) were contained as other components | | | | | | |

**[Table 3-2]**

| component* | content (w/v%) | | | | | | |
|---|---|---|---|---|---|---|---|
| | Ex. 3 | Ex. 4 | Ex. 5 | Ex. 6 | Ex. 7 | Ex. 8 | Ex. 9 |
| Compound A | 0.0002 | 0.0010 | 0.005 | 0.05 | 0.0005 | 0.0005 | 0.0005 |
| tyloxapol | 0.10 | 0.10 | 0.10 | 0.50 | 0.01 | 0.05 | 0.50 |
| pH | 7.5 | 7.5 | 7.5 | 7.5 | 7.5 | 7.5 | 7.5 |
| residual ratio (%) of compound A after storage at 80°C for 1 week | 79.0 | 80.6 | 84.2 | 93.0 | 73.7 | 76.5 | 98.0 |

| | | | | | | | |
|---|---|---|---|---|---|---|---|
| *: sodium dihydrogen phosphate (0.1 w/v%), sodium hydroxide/hydrochloric acid (q.s.) and purified water (q.s.) were contained as other components | | | | | | | |

### (Test results)

When tyloxapol or octoxynol was added, the stability of compound A in the aqueous solution after storage at 80°C for 1 week could be maintained remarkably high (Table 3-1, Table 3-2) as compared to other preparations (preparations of Comparative Example 1, Comparative Example 2 and Comparative Example 3 of Table 3-1).

From the results of Experimental Example 1 and Experimental Example 2, it was found that addition of tyloxapol or octoxynol is preferable for securing stability of compound A to light and heat in an aqueous solution.

[Experimental Example 3] Light stability and heat stability of compound A in aqueous solution by the addition of alcohol

### (Test method)

An aqueous solution of compound A was prepared according to the following formulation. To 0.1% phosphate buffer was added tyloxapol (0.1 w/v%), and the mixture was adjusted to pH 7.5 with sodium hydroxide. A predetermined amount of alcohol was added and dissolved therein. Compound A in the predetermined amount in the following formulation was dissolved in said solution, and the mixture was sterilized by filtration with a 0.22 µm membrane filter, and filled in a 5 mL colorless glass ampoule. The glass ampoule was placed under light irradiation conditions and storage conditions at 80°C for 1 week, and the content of each compound A was measured. Compound A was quantified in the same manner as in Experimental Example 1 and Experimental Example 2.

**[Table 4-1]**

| component* | content (w/v%) | | | | | | | | | |
|---|---|---|---|---|---|---|---|---|---|---|
| | Ref. Ex. | Ex.1 | Ex. 10 | Ex. 11 | Ex. 12 | Ex. 13 | Comp. Ex. 4 | Comp. Ex. 5 | Comp. Ex. 6 | Comp. Ex. 7 |
| Compound A | 0.0005 | 0.0005 | 0.0005 | 0.0005 | 0.0005 | 0.0005 | 0.0005 | 0.0005 | 0.0005 | 0.0005 |
| concentrated glycerin | - | - | 2.40 | - | - | - | 2.40 | - | - | - |
| D-mannitol | - | - | - | 4.60 | - | - | - | 4.60 | 4.60 - | - |
| propylene glycol | - | - | - | - | 1.00 | - | - | - | 1.00 | - |
| ethanol | - | - | - | - | - | 0.5 | - | - | - | 0.5 |
| tyloxapol | - | 0.10 | 0.10 | 0.10 | 0.10 | 0.10 | - | - | - | - |
| pH | 7.5 | 7.5 | 7.5 | 7.5 | 7.5 | 7.5 | 7.5 | 7.5 | 7.5 | 7.5 |
| residual ratio (%) after light irradiation (12000 lux·h) | 70.3 | 92.6 | 92.5 | 92.0 | 93.1 | 92.9 | 67.3 | 67.0 | 67.5 | 68.2 |
| residual ratio (%) after light irradiation (24000 lux·h) | 46.0 | 86.9 | 86.9 | 87.4 | 85.9 | 87.4 | 46.5 | 46.9 | 44.4 | 48.4 |
| residual ratio (%) of compound A after storage at 80°C for 1 week | 98.5 | 90.3 | 100.4 | 101.2 | 101. 2 | 96.6 | 100.6 | 101.8 | 102.1 | 102.8 |

| | | | | | | | | | | |
|---|---|---|---|---|---|---|---|---|---|---|
| *: sodium dihydrogen phosphate (0.1 w/v%), sodium hydroxide/hydrochloric acid (q.s.) and purified water (q.s.) were contained as other components | | | | | | | | | | |

**[Table 4-2]**

| component* | content (w/v%) | | |
|---|---|---|---|
| | Example 14 | Example 15 | Example 16 |
| Compound A | 0.0002 | 0.005 | 0.05 |
| propylene glycol | 1.00 | 1.00 | 1.00 |
| tyloxapol | 0.10 | 0.10 | 0.50 |
| pH | 7.5 | 7.5 | 7.5 |
| residual ratio (%) after light irradiation (12000 lux-h) | 92.5 | 97.2 | 99.2 |
| residual ratio (%) after light irradiation (24000 lux·h) | 84.3 | 94.7 | 98.9 |
| residual ratio (%) of compound A after storage at 80°C for 1 week | 96.5 | 97.0 | 97.8 |

| | | | |
|---|---|---|---|
| *: sodium dihydrogen phosphate (0.1 w/v%), sodium hydroxide/hydrochloric acid (q.s.) and purified water (q.s.) were contained as other components | | | |

### (Test results)

Regardless of the kind of alcohol, preparations concurrently added with tyloxapol and alcohol could further improve the heat stabilizing effect without preventing the light stabilizing effect of compound A (Examples 1 and 10 - 13 of Table 4-1 and Examples 14 - 16 of Table 4-2). On the other hand, preparations free of tyloxapol showed remarkably decreased light stability of compound A in the aqueous solution, even though alcohol was added (Comparative Examples 4 - 7 and Reference Example of Table 4-1). Regardless of the content of compound A, moreover, a heat stability improving effect by the addition of alcohol could be confirmed (Example 12 of Table 4-1 and Examples 14 - 16 of Table 4-2).

### [Formulation Examples]

According to the formulations shown in Table 5-1 - Table 5-5, compound A-containing ophthalmic solutions were prepared according to a conventional method (Formulation Examples 1 - 37).

**[Table 5-1]**

| component·content (w/v%) | Formulation Example 1 | Formulation Example 2 | Formulation Example 3 | Formulation Example 4 | Formulation Example 5 | Formulation Example 6 | Formulation Example 7 | Formulation Example 8 |
|---|---|---|---|---|---|---|---|---|
| Compound A | 0.0005 | 0.0005 | 0.0005 | 0.0005 | 0.0002 | 0.0002 | 0.0002 | 0.0002 |
| sodium dihydrogen phosphate | 0.1 | 0.1 | 0.1 | 0.1 | 0.1 | 0.1 | 0.1 | 0.1 |
| tyloxapol | 0.1 | 0.1 | 0.1 | 0.1 | 0.1 | 0.1 | 0.1 | 0.1 |
| concentrated glycerin | 2.4 | - | - | - | 2.4 | - | - | - |
| D-mannitol | - | 4.6 | - | - | - | 4.6 | - | - |
| propylene glycol | - | - | 1.0 | - | - | - | 1.0 | - |
| ethanol | - | - | - | 0.5 | - | - | - | 0.5 |
| sodium hydroxide | q.s. | q.s. | q.s. | q.s. | q.s. | q.s. | q.s. | q.s. |
| hydrochloric acid | q.s. | q.s. | q.s. | q.s. | q.s. | q.s. | q.s. | q.s. |
| benzalkonium chloride | 0.005 | 0.005 | 0.005 | 0.005 | 0.003 | 0.003 | 0.003 | 0.003 |
| purified water | q.s. | q.s. | q.s. | q.s. | q.s. | q.s. | q.s. | q.s. |
| total amount (mL) | 100 | 100 | 100 | 100 | 100 | 100 | 100 | 100 |
| pH | 7.5 | 7.5 | 7.5 | 7.5 | 7.5 | 7.5 | 7.5 | 7.5 |

**[Table 5-2]**

| component-content (w/v%) | Formulation Example 9 | Formulation Example 10 | Formulation Example 11 | Formulation Example 12 | Formulation Example 13 | Formulation Example 14 | Formulation Example 15 | Formulation Example 16 |
|---|---|---|---|---|---|---|---|---|
| Compound A | 0.001 | 0.001 | 0.001 | 0.001 | 0.005 | 0.005 | 0.005 | 0.005 |
| sodium dihydrogen phosphate | 0.1 | 0.1 | 0.1 | 0.1 | 0.1 | 0.1 | 0.1 | 0.1 |
| tyloxapol | 0.1 | 0.1 | 0.1 | 0.1 | 0.1 | 0.1 | 0.1 | 0.1 |
| concentrated glycerin | 2.4 | - | - | - | 2.4 | - | - | - |
| D-mannitol | - | 4.6 | - | - | - | 4.6 | - | - |
| propylene glycol | - | - | 1.0 | - | - | - | 1.0 | - |
| ethanol | - | - | - | 0.5 | - | - | - | 0.5 |
| sodium hydroxide | q.s. | q.s. | q.s. | q.s. | q.s. | q.s. | q.s. | q.s. |
| hydrochloric acid | q.s. | q.s. | q.s. | q.s. | q.s. | q.s. | q.s. | q.s. |
| benzalkonium chloride | 0.005 | 0.005 | 0.005 | 0.005 | 0.005 | 0.005 | 0.005 | 0.005 |
| purified water | q.s. | q.s. | q.s. | q.s. | q.s. | q.s. | q.s. | q.s. |
| total amount (mL) | 100 | 100 | 100 | 100 | 100 | 100 | 100 | 100 |
| pH | 7.5 | 7.5 | 7.5 | 7.5 | 7.5 | 7.5 | 7.5 | 7.5 |

**[Table 5-3]**

| component-content (w/v%) | Formulation Example 17 | Formulation Example 18 | Formulation Example 19 | Formulation Example 20 | Formulation Example 21 | Formulation Example 22 | Formulation Example 23 | Formulation Example 24 |
|---|---|---|---|---|---|---|---|---|
| Compound A | 0.05 | 0.05 | 0.05 | 0.05 | 0.1 | 0.1 | 0.1 | 0.1 |
| sodium dihydrogen phosphate | 0.1 | 0.1 | 0.1 | 0.1 | 0.1 | 0.1 | 0.1 | 0.1 |
| tyloxapol | 0.5 | 0.5 | 0.5 | 0.5 | 0.5 | 0.5 | 0.5 | 0.5 |
| concentrated glycerin | 2.4 | - | - | - | 2.4 | - | - | - |
| D-mannitol | - | 4.6 | - | - | - | 4.6 | - | - |
| propylene glycol | - | - | 1.0 | - | - | - | 1.0 | - |
| ethanol | - | - | - | 0.5 | - | - | - | 0.5 |
| sodium hydroxide | q.s. | q.s. | q.s. | q.s. | q.s. | q.s. | q.s. | q.s. |
| hydrochloric acid | q.s. | q.s. | q.s. | q.s. | q.s. | q.s. | q.s. | q.s. |
| benzalkonium chloride | 0.005 | 0.005 | 0.005 | 0.005 | 0.005 | 0.005 | 0.005 | 0.005 |
| purified water | q.s. | q.s. | q.s. | q.s. | q.s. | q.s. | q.s. | q.s. |
| total amount (mL) | 100 | 100 | 100 | 100 | 100 | 100 | 100 | 100 |
| pH | 7.5 | 7.5 | 7.5 | 7.5 | 7.5 | 7.5 | 7.5 | 7.5 |

**[Table 5-4]**

| component· content (w/v%) | Formulation Example 25 | Formulation Example 26 | Formulation Example 27 | Formulation Example 28 | Formulation Example 29 | Formulation Example 30 |
|---|---|---|---|---|---|---|
| Compound A | 0.0005 | 0.0002 | 0.001 | 0.005 | 0.05 | 0.1 |
| boric acid | 1.0 | 1.0 | 1.0 | 1.0 | 1.0 | 1.0 |
| borax | q.s. | q.s. | q.s. | q.s. | q.s. | q.s. |
| tyloxapol | 0.1 | 0.1 | 0.1 | 0.1 | 0.5 | 0.5 |
| concentrated glycerin | 2.4 | 2.4 | 2.4 | 2.4 | 2.4 | 2.4 |
| sodium hydroxide | q.s. | q.s. | q. s. | q.s. | q.s. | q.s. |
| hydrochloric acid | q.s. | q.s. | q.s. | q.s. | q.s. | q.s. |
| chlorhexidine gluconate | 0.005 | 0.003 | 0.005 | 0.005 | 0.005 | 0.005 |
| purified water | q.s. | q.s. | q.s. | q.s. | q.s. | q.s. |
| total amount (mL) | 100 | 100 | 100 | 100 | 100 | 100 |
| pH | 7.5 | 7.5 | 7.5 | 7.5 | 7.5 | 7.5 |

**[Table 5-5]**

| component-content (w/v%) | Formulation Example 31 | Formulation Example 32 | Formulation Example 33 | Formulation Example 34 | Formulation Example 35 | Formulation Example 36 | Formulation Example 37 |
|---|---|---|---|---|---|---|---|
| compound A | 0.0005 | 0.001 | 0.005 | 0.05 | 0.001 | 0.005 | 0.05 |
| boric acid | - | 1.0 | 1.0 | 1.0 | 1.0 | 1.0 | 1.0 |
| borax | - | q.s. | q.s. | q.s. | q.s. | q.s. | q.s. |
| sodium dihydrogen phosphate | 0.1 | - | - | - | - | - | - |
| tyloxapol | - | 0.1 | 0.1 | 0.1 | - | - | - |
| octoxynol | 0.1 | - | - | - | 0.1 | 0.1 | 0.1 |
| propylene glycol | 1.0 | 1.0 | 1.0 | 1.0 | 1.0 | 1.0 | 1.0 |
| sodium hydroxide | q.s. | q.s. | q.s. | q.s. | q.s. | q.s. | q.s. |
| hydrochloric acid | q.s. | q.s. | q.s. | q.s. | q.s. | q.s. | q.s. |
| benzalkonium chloride | 0.005 | 0.005 | 0.005 | 0.005 | 0.005 | 0.005 | 0.005 |
| purified water | q.s. | q.s. | q.s. | q.s. | q.s. | q.s. | q.s. |
| total amount (mL) | 100 | 100 | 100 | 100 | 100 | 100 | 100 |
| pH | 7.5 | 7.5 | 7.5 | 7.5 | 7.5 | 7.5 | 7.5 |

### Industrial Applicability

According to the present invention, in an aqueous liquid preparation containing compound A useful as a therapeutic agent for ocular diseases such as meibomian gland dysfunction, corneal epithelial disorder, dry eye and the like, the stability of compound A to light and heat in the aqueous liquid preparation can be remarkably improved by adding tyloxapol or octoxynol, and an aqueous liquid preparation wherein solubility of compound A is also improved can be provided.

## Claims

1. An aqueous liquid preparation comprising (3-{2-[4-isopropyl-2-(4-trifluoromethyl)phenyl-5-thiazolyl]ethyl}-5-methyl-1,2-benzisoxazol-6-yl)oxyacetic acid or a pharmaceutically acceptable salt thereof, and tyloxapol or octoxynol.

2. An aqueous liquid preparation according to claim 1 comprising (3-{2-[4-isopropyl-2-(4-trifluoromethyl)phenyl-5-thiazolyl]ethyl}-5-methyl-1,2-benzisoxazol-6-yl)oxyacetic acid or a pharmaceutically acceptable salt thereof, and tyloxapol.

3. An aqueous liquid preparation according to claim 1 comprising (3-{2-[4-isopropyl-2-(4-trifluoromethyl)phenyl-5-thiazolyl]ethyl}-5-methyl-1,2-benzisoxazol-6-yl)oxyacetic acid or a pharmaceutically acceptable salt thereof, and octoxynol.

4. The aqueous liquid preparation according to any one of claims 1 to 3, wherein (3-{2-[4-isopropyl-2-(4-trifluoromethyl)phenyl-5-thiazolyl]ethyl}-5-methyl-1,2-benzisoxazol-6-yl)oxyacetic acid or a pharmaceutically acceptable salt thereof has a concentration selected from the range of the lower limit concentration of 0.0002 w/v% and the upper limit concentration of 0.05 w/v% relative to the total amount of the aqueous liquid preparation.

5. The aqueous liquid preparation according to claim 1, 2 or 4, wherein tyloxapol has a concentration selected from the range of the lower limit concentration of 0.01 w/v% and the upper limit concentration of 0.5 w/v% relative to the total amount of the aqueous liquid preparation.

6. The aqueous liquid preparation according to claim 1, 3 or 4, wherein octoxynol has a concentration selected from the range of the lower limit concentration of 0.01 w/v% and the upper limit concentration of 0.5 w/v% relative to the total amount of the aqueous liquid preparation.

7. The aqueous liquid preparation according to any one of claims 1 to 6, further comprising alcohol.

8. The aqueous liquid preparation according to claim 7, wherein alcohol has a concentration selected from the range of the lower limit concentration of 0.1 w/v% and the upper limit concentration of 5 w/v% relative to the total amount of the aqueous liquid preparation.

9. The aqueous liquid preparation according to claim 7 or 8, wherein alcohol comprises at least any selected from glycerin, sugar alcohol, glycol and ethanol.

10. The aqueous liquid preparation according to claim 7 or 8, wherein alcohol comprises at least any selected from glycerin, mannitol, propylene glycol and ethanol.

11. The aqueous liquid preparation according to claim 7 or 8, wherein alcohol comprises propylene glycol.

12. The aqueous liquid preparation according to any one of claims 1 to 11, which has transmittance at wavelength 600 nm of not less than 98%.

13. The aqueous liquid preparation according to any one of claims 1 to 12, which is for ophthalmology.

14. The aqueous liquid preparation according to claim 13, which is an ophthalmic solution.

15. Use of tyloxapol or octoxynol in an aqueous liquid preparation of any one of claims 1 to 12 comprising (3-{2-[4-isopropyl-2-(4-trifluoromethyl)phenyl-5-thiazolyl]ethyl}-5-methyl-1,2-benzisoxazol-6-yl)oxyacetic acid or a pharmaceutically acceptable salt thereof, for stabilizing the (3-{2-[4-isopropyl-2-(4-trifluoromethyl)phenyl-5-thiazolyl]ethyl}-5-methyl-1,2-benzisoxazol-6-yl)oxyacetic acid or a pharmaceutically acceptable salt thereof in the aqueous liquid preparation to light and heat.

16. A method of stabilizing (3-{2-[4-isopropyl-2-(4-trifluoromethyl)phenyl-5-thiazolyl]ethyl}-5-methyl-1,2-benzisoxazol-6-yl)oxyacetic acid or a pharmaceutically acceptable salt thereof to light and heat in an aqueous liquid preparation, comprising adding tyloxapol or octoxynol to an aqueous liquid preparation comprising (3-{2-[4-isopropyl-2-(4-trifluoromethyl)phenyl-5-thiazolyl]ethyl}-5-methyl-1,2-benzisoxazol-6-yl)oxyacetic acid or a pharmaceutically acceptable salt thereof, thereby to produce an aqueous liquid preparation in accordance with any one of claims 1 to 12.

## Patentansprüche

1. Wässriges flüssiges Präparat, das (3-{2-[4-Isopropyl-2-(4-trifluormethyl)-phenyl-5-thiazolyl]ethyl}-5-methyl-1,2-benzisoxazol-6-yl)oxyessigsäure oder ein pharmazeutisch annehmbares Salz davon sowie Tyloxapol oder Octoxynol umfasst.

2. Wässriges flüssiges Präparat gemäß Anspruch 1, das (3-{2-[4-Isopropyl-2-(4-trifluormethyl)phenyl-5-thiazolyl]ethyl}-5-methyl-1,2-benzisoxazol-6-yl)oxyessigsäure oder ein pharmazeutisch annehmbares Salz davon sowie Tyloxapol umfasst.

3. Wässriges flüssiges Präparat gemäß Anspruch 1, das (3-{2-[4-Isopropyl-2-(4-trifluormethyl)phenyl-5-thiazolyl]ethyl}-5-methyl-1,2-benzisoxazol-6-yl)oxyessigsäure oder ein pharmazeutisch annehmbares Salz davon sowie Octoxynol umfasst.

4. Wässriges flüssiges Präparat gemäß einem der Ansprüche 1 bis 3, wobei (3-{ 2-[4-Isopropyl-2-(4-trifluormethyl)phenyl-5-thiazolyl]ethyl} -5-methyl-1,2-benzisoxazol-6-yl)oxyessigsäure oder ein pharmazeutisch annehmbares Salz davon eine Konzentration aufweist, die aus dem Bereich der unteren Grenzkonzentration von 0,0002% (w/v) und der oberen Grenzkonzentration von 0,05% (w/v) ausgewählt ist, bezogen auf die Gesamtmenge des wässrigen flüssigen Präparats.

5. Wässriges flüssiges Präparat gemäß Anspruch 1, 2 oder 4, wobei Tyloxapol eine Konzentration aufweist, die aus dem Bereich der unteren Grenzkonzentration von 0,01% (w/v) und der oberen Grenzkonzentration von 0,5% (w/v) ausgewählt ist, bezogen auf die Gesamtmenge des wässrigen flüssigen Präparats.

6. Wässriges flüssiges Präparat gemäß Anspruch 1, 3 oder 4, wobei Octoxynol eine Konzentration aufweist, die aus dem Bereich der unteren Grenzkonzentration von 0,01% (w/v) und der oberen Grenzkonzentration von 0,5% (w/v) ausgewählt ist, bezogen auf die Gesamtmenge des wässrigen flüssigen Präparats.

7. Wässriges flüssiges Präparat gemäß einem der Ansprüche 1 bis 6, das weiterhin Alkohol umfasst.

8. Wässriges flüssiges Präparat gemäß Anspruch 7, wobei Alkohol eine Konzentration aufweist, die aus dem Bereich der unteren Grenzkonzentration von 0,1% (w/v) und der oberen Grenzkonzentration von 5% (w/v) ausgewählt ist, bezogen auf die Gesamtmenge des wässrigen flüssigen Präparats.

9. Wässriges flüssiges Präparat gemäß Anspruch 7 oder 8, wobei Alkohol wenigstens welche umfasst, die aus Glycerin, Zuckeralkohol, Glycol und Ethanol ausgewählt sind.

10. Wässriges flüssiges Präparat gemäß Anspruch 7 oder 8, wobei Alkohol wenigstens welche umfasst, die aus Glycerin, Mannit, Propylenglycol und Ethanol ausgewählt sind.

11. Wässriges flüssiges Präparat gemäß Anspruch 7 oder 8, wobei Alkohol Propylenglycol umfasst.

12. Wässriges flüssiges Präparat gemäß einem der Ansprüche 1 bis 11, das eine Transmission bei einer Wellenlänge von 600 nm von nicht weniger als 98% aufweist.

13. Wässriges flüssiges Präparat gemäß einem der Ansprüche 1 bis 12, das für die Augenheilkunde bestimmt ist.

14. Wässriges flüssiges Präparat gemäß Anspruch 13, bei dem es sich um Augentropfen handelt.

15. Verwendung von Tyloxapol oder Octoxynol in einem wässrigen flüssigen Präparat gemäß einem der Ansprüche 1 bis 12, das (3-{2-[4-Isopropyl-2-(4-trifluormethyl)phenyl-5-thiazolyl]ethyl}-5-methyl-1,2-benzisoxazol-6-yl)oxyessigsäure oder ein pharmazeutisch annehmbares Salz davon umfasst, zum Stabilisieren der (3-{2-[4-Isopropyl-2-(4-trifluormethyl)-phenyl-5-thiazolyl]ethyl}-5-methyl-1,2-benzisoxazol-6-yl)oxyessigsäure oder ihres pharmazeutisch annehmbaren Salzes in dem wässrigen flüssigen Präparat gegenüber Licht und Wärme.

16. Verfahren zum Stabilisieren von (3-{2-[4-Isopropyl-2-(4-trifluormethyl)-phenyl-5-thiazolyl]ethyl}-5-methyl-1,2-benzisoxazol-6-yl)oxyessigsäure oder eines pharmazeutisch annehmbaren Salzes davon gegenüber Licht und Wärme in einem wässrigen flüssigen Präparat, umfassend das Hinzufügen von Tyloxapol oder Octoxynol zu einem wässrigen flüssigen Präparat, das (3-{2-[4-Isopropyl-2-(4-trifluormethyl)phenyl-5-thiazolyl]ethyl}-5-methyl-1,2-benzisoxazol-6-yl)oxyessigsäure oder ein pharmazeutisch annehmbares Salz davon umfasst, wodurch ein wässriges flüssiges Präparat gemäß einem der Ansprüche 1 bis 12 entsteht.

## Revendications

1. Préparation liquide aqueuse comprenant de l'acide (3-{2-[4-isopropyl-2-(4-trifluorométhyl)phényl-5-thiazolyl]éthyl}-5-méthyl-1,2-benzisoxazol-6-yl)oxyacétique ou l'un de ses sels pharmaceutiquement acceptables, et du tyloxapol ou de l'octoxynol.

2. Préparation liquide aqueuse selon la revendication 1 comprenant de l'acide (3-{2-[4-isopropyl-2-(4-trifluorométhyl)phényl-5-thiazolyl]-éthyl}-5-méthyl-1,2-benzisoxazol-6-yl)oxyacétique ou l'un de ses sels pharmaceutiquement acceptables, et du tyloxapol.

3. Préparation liquide aqueuse selon la revendication 1 comprenant de l'acide (3-{2-[4-isopropyl-2-(4-trifluorométhyl)phényl-5-thiazolyl]-éthyl}-5-méthyl-1,2-benzisoxazol-6-yl)oxyacétique ou l'un de ses sels pharmaceutiquement acceptables, et de l'octoxynol.

4. Préparation liquide aqueuse selon l'une quelconque des revendications 1 à 3, dans laquelle l'acide (3-{2-[4-isopropyl-2-(4-trifluorométhyl)phényl-5-thiazolyl]éthyl}-5-méthyl-1,2-benzisoxazol-6-yl)oxyacétique ou l'un de ses sels pharmaceutiquement acceptables a une concentration choisie dans la plage de la concentration limite inférieure de 0,0002 % p/v et de la concentration limite supérieure de 0,05 % p/v par rapport à la quantité totale de la préparation liquide aqueuse.

5. Préparation liquide aqueuse selon la revendication 1, 2 ou 4, dans laquelle le tyloxapol a une concentration choisie dans la plage de la concentration limite inférieure de 0,01 % p/v et de la concentration limite supérieure de 0,5 % p/v par rapport à la quantité totale de la préparation liquide aqueuse.

6. Préparation liquide aqueuse selon la revendication 1, 3 ou 4, dans laquelle l'octoxynol a une concentration choisie dans la plage de la concentration limite inférieure de 0,01 % p/v et de la concentration limite supérieure de 0,5 % p/v par rapport à la quantité totale de la préparation liquide aqueuse.

7. Préparation liquide aqueuse selon l'une quelconque des revendications 1 à 6, comprenant en outre de l'alcool.

8. Préparation liquide aqueuse selon la revendication 7, dans laquelle l'alcool a une concentration choisie dans la plage de la concentration limite inférieure de 0,1 % p/v et de la concentration limite supérieure de 5 % p/v par rapport à la quantité totale de la préparation liquide aqueuse.

9. Préparation liquide aqueuse selon la revendication 7 ou 8, dans laquelle l'alcool comprend au moins l'un quelconque choisi parmi la glycérine, un alcool de sucre, un glycol et l'éthanol.

10. Préparation liquide aqueuse selon la revendication 7 ou 8, dans laquelle l'alcool comprend au moins l'un quelconque choisi parmi la glycérine, le mannitol, le propylène glycol et l'éthanol.

11. Préparation liquide aqueuse selon la revendication 7 ou 8, dans laquelle l'alcool comprend du propylène glycol.

12. Préparation liquide aqueuse selon l'une quelconque des revendications 1 à 11, qui présente une transmittance à la longueur d'onde de 600 nm d'au moins 98 %.

13. Préparation liquide aqueuse selon l'une quelconque des revendications 1 à 12, qui est destinée à l'ophtalmologie.

14. Préparation liquide aqueuse selon la revendication 13, qui est une solution ophtalmique.

15. Utilisation de tyloxapol ou d'octoxynol dans une préparation liquide aqueuse selon l'une quelconque des revendications 1 à 12 comprenant de l'acide (3-{2-[4-isopropyl-2-(4-trifluorométhyl)phényl-5-thiazolyl]-éthyl}-5-méthyl-1,2-benzisoxazol-6-yl)oxyacétique ou l'un de ses sels pharmaceutiquement acceptables, pour la stabilisation de l'acide (3-{2-[4-isopropyl-2-(4-trifluorométhyl)phényl-5-thiazolyl]éthyl}-5-méthyl-1,2-benzisoxazol-6-yl)oxyacétique ou de l'un de ses sels pharmaceutiquement acceptables dans la préparation liquide aqueuse à la lumière et à la chaleur.

16. Procédé de stabilisation de l'acide (3-{2-[4-isopropyl-2-(4-trifluorométhyl)phényl-5-thiazolyl]-éthyl}-5-méthyl-1,2-benzisoxazol-6-yl)oxyacétique ou de l'un de ses sels pharmaceutiquement acceptables à la lumière et à la chaleur dans une préparation liquide aqueuse, comprenant l'addition de tyloxapol ou d'octoxynol à une préparation liquide aqueuse comprenant de l'acide (3-{2-[4-isopropyl-2-(4-trifluoro-méthyl)phényl-5-thiazolyl]éthyl}-5-méthyl-1,2-benzisoxazol-6-yl)oxyacétique ou l'un de ses sels pharmaceutiquement acceptables, produisant de cette façon une préparation liquide aqueuse conformément à l'une quelconque des revendications 1 à 12.
